# EUROPEAN PATENT SPECIFICATION

(11) **EP 2 591 768 B1**
(45) Date of publication and mention of the grant of the patent: **16.07.2014**
(21) Application number: 11188919.2
(22) Date of filing: 14.11.2011
(51) Int. Cl.: A61K 9/00, A61K 9/16, A61K 9/20, A61K 31/593, A61K 31/785, A61K 47/02, A61K 47/20, A61K 47/38, A61K 47/44, A61K 9/24, A61K 31/07, A61K 31/122, A61K 31/355, A61K 31/59

(54) **Single unit dosage formulations of sevelamer and fat soluble vitamins and surface active agents**
Einzeldosisformulierungen von Sevelamer und fettlöslichen Vitaminen und oberflächenaktiven Substanzen
Formulations de dose unitaire de sevelamer et des vitamines liposolubles et des tensioactifs

(43) Date of publication of application: 15.05.2013
(73) Proprietor: Deva Holding Anonim Sirketi, 34303 Istanbul (TR)
(72) Inventor: Haas, Philipp Daniel, 34303 Istanbul (TR); Koc, Fikret, 34303 Istanbul (TR); Sivasligil, Ramazan, 34303 Istanbul (TR); Kandemir, Levent, 34303 Istanbul (TR)

(56) References cited:
- WO-A2-02/09815
- WO-A2-2011/135591
- SLATOPOLSKY E A ET AL: "RENAGEL, A NONABSORBED CALCIUM- AND ALUMINUM-FREE PHOSPHATE BINDER, LOWERS SERUM PHOSPHORUS AND PARATHYROID HORMONE", KIDNEY INTERNATIONAL, NATURE PUBLISHING GROUP, LONDON, GB, vol. 55, no. 1, 1 January 1999 (1999-01-01), pages 299-307, XP008049273, ISSN: 0085-2538, DOI: 10.1046/J.1523-1755.1999.00240.X
- PAWEENA SUSANTITAPHONG ET AL: "Potential Interaction Between Sevelamer and Fat-Soluble Vitamins: A Hypothesis", AMERICAN JOURNAL OF KIDNEY DISEASES, vol. 59, no. 2, 1 February 2012 (2012-02-01), pages 165-167, XP55024578, ISSN: 0272-6386, DOI: 10.1053/j.ajkd.2011.12.001

## Description

### Technical Field

The invention relates to pharmaceutical formulations comprising sevelamer or a pharmaceutically acceptable salt thereof and a fat soluble vitamin which is selected from the group consisting of A, D, E or K or analogues or mixtures thereof in combination in oral solid dosage formulations as well as methods of manufacturing of monolithic tablet, bilayer tablet, tablet in tablet, in lay tablet, granules in sachet, coated core tablet forms by achieving targeted content uniformity and patient compliance.

### Background Art

Chronic kidney disease is an emerging public health problem and one of precursor of premature cardiovascular disease. In patients with end stage renal disease, hyperphosphatemia is a common condition and is typically defined for humans as a serum phosphate level of greater than about 4.5 mg/ dL.

Phosphate binding polymers are indicated for treatment of hyperphosphatemia in patients undergoing haemodialysis for end stage renal failure for removing phosphate from gastrointestinal tract.

Hyperphosphatemia is often manifested by hyperparathyroidism and may significantly lower the concentration of serum calcium as well. To minimize the risk of hypercalcemia and excess Ca²⁺ burden, clinicians are increasingly relying on Ca²⁺ free, PO₄³-binder such as sevelamer as salt of HCl or carbonate. Sevelamer lowers the phosphate concentration in serum by binding phosphate in the gastrointestinal tract, and indicated for the control of hyperphosphatemia in adult haemodialysis patient. Sevelamer HCl is currently being marketed as RENAGEL® and sevelamer carbonate is currently being marketed as RENVELA® for the treatment of patients with chronic kidney disease which are on haemodialysis.

Sevelamer HCl is non absorbed phosphate binding polyallylamine hydrochloride crosslinked with epichlorohydrin in which 40% of the amines are protonated. Sevelamer is hydrophilic and swells but insoluble in water.

Sevelamer HCl is free of aluminium and calcium which is used to prevent and treat high blood phosphate levels of and allow controlling serum phosphorus without elevation of serum Ca, Mg, and Al in patients with end stage renal disease.

If present over extended periods of time without treatment, hyperphosphatemia may lead to severe abnormalities in calcium and phosphorus metabolism. Consequently, patients with chronic kidney disease (CKD) often require oral vitamin D supplementation (25-hydroxyvitamin d) as well as treatment with phosphate binders as of the inception of the chronic kidney disease.

Sevelamer may lead to decreased absorption of fat soluble vitamins such as vitamin D. Depending on the diet intake and the nature of the end stage renal failure, dialysis patients may develop low vitamin A, D, E, K levels.

Vitamin D is metabolized in the liver to circulating form of 25-hydroxyvitamin D which determines a patient's vitamin d status and hereafter to active form 1, 25- dihydroxyvitamin D in the kidney. Nevermore, hyperphosphatemia decreases the activity of the enzyme responsible for converting inactive vitamin D to active vitamin D and patients with CKD have a severe vitamin D deficiency that has reduced ability to convert 25-hydroxyvitamin D into the active form 1,25 dihydroxyvitamin D.

Vitamin D deficiency is assessed by level of 25- hydroxyvitamin D (25 OHD). 25-OHD insufficiency is defined as 25-OHD of less than 50 nmol/Liter or 20 ng/mliter. The optimal range of serum (25-OHD) is assumed to be 32-50 ng /ml or higher.

Efficacy of cholecalciferol (vitamin D₃) therapy in correcting vitamin D insufficiency patients with chronic renal disease has been proved by clinical studies.

25-OHD exists in 2 distinct forms; 25-hydroxyergocalciferol (25OHD₂) and 25-hydroxycholecalciferol (25 OHD₃). National Kidney Foundation recommends that, in patients with chronic kidney disease, 25-hydroxyvitamin D should be measured regularly, the level should be maintained at 30 ng per millilitre.

**_**SLATOPOLSKY, EDUARDO A., et al. RenaGel, a nonabsorbed calcium-and aliminium free phosphate binder,lowers serum phosphorus and parathyroid hormone. Kidney International. 1999, vol.55, p.299-307. discloses co administration sevelamer and vitamin D, it does not contain single unit dose of sevelamer and vitamin D.

PCT/WO 2011135591 A (SHASUN PHARMACEUTICALS LIMITED) 11/3/2011 denotes pharmaceutical composition consisting sevelamer and surface active agent as well as other ingredients in which Vitamin D is not presence._

PCT/WO 0209815 A (F.HOFFMANN-LA ROCHE AG) 2/7/2002 discloses a pharmaceutical composition comprising a lipase inhibitor and a pharmaceutically acceptable bile acid sequestrant wherein. It is not directed to single unit dose of sevelamer and vitamin D

SUSANTITAPHONG, PAWEENA, et al. POTENTIAL INTERACTION BETWEEN SE VELAMER AND FAT SOLUBLE VITAMINS:A HYPOTHESIS. AM.J.KIDNEY. 2012, vol.59, no.2, p.165-167. Discloses using of sevelamer with vitamin D or vitamin K. It does not assert single unit dose of sevelamer and vitamin D or vitamin K._

### Summary of invention

The present invention is related to enhanced solid oral fixed dose combinations of formulations comprising sevelamer or a pharmaceutically acceptable salt thereof and a fat soluble vitamin which is selected from the group consisting of A, D, E or K or analogues or mixtures thereof whereby vitamin or vitamins or analogues thereof are co-formulated with a surface active agent to improve content uniformity in single dosage form and patient compliance upon monolithic tablet, bilayer tablet, tablet in tablet, inlay tablet, granules in sachets and coated core tablet formulations.

### Technical Problem

In manufacturing of solid oral dosage forms, content uniformity is an important parameter to consider especially in combination formulations containing two active pharmaceutical ingredients wherein doses of these are significantly different from each other.

Solid oral dosage forms of sevelamer or a pharmaceutically acceptable salt thereof and a fat soluble vitamin selected from group consisting of A, D, E, K or analogues or mixtures of them with acceptable content homogeneity require careful formulation and development of production process.

In the present invention, the therapeutic dose of sevelamer is very high. The marketed dosage forms of sevelamer comprise 400 mg or 800 mg of the active substance per tablet or 800 mg or 2.4 g powder per sachet for liquid reconstitution. The daily dose may namely be up to 14 g per day depending on the severity of disease.

The Institute of Medicine (IOM) of the National Academy of Science has declared that the adequate intake of Vitamin D for healthy individual ranges from 200 to 600 IU per day, but vitamin D supplementation above this value is frequently needed for achieving and maintaining optimal blood 25-hydroxyvitamin D levels in CRD patients. As for vitamin D deficiency treatment, some clinical trials suggest administration of vitamin D of 50,000 IU once a week for 12 weeks. When calculated in terms of microgram, above mentioned quantities of Vitamin D vary from 5 mcg to 1,250 mcg per dosage. Same situation is also valid for vitamins of A, E and K.

Even at higher dose than the recommended dose, micro dosing of fat soluble vitamin selected from the group consisting of A, D, E or K or analogues or mixtures of them is difficult to distribute uniformly both in combination with other drugs or alone throughout solid oral fixed pharmaceutical dosage forms.

Active vitamin A, D, E or K compounds or analogues or mixtures of them are lipophilic, they are soluble in lipids and some organic solvents, while being insoluble or sparsely soluble in water. Because of the dispersion of said compounds is limited in aqueous solutions, formulation and production of finished dosage forms of containing vitamin A, D, E or K or their analogues or mixtures of them are both very complicated.

Additionally, in chronic renal failure drug treatment including sevelamer and a vitamin A, D, E or K or their analogues or mixtures of them, multiple medications must be taken in a number of times throughout the day. Taking separately sevelamer and a vitamin A, D, E or K or their analogues or mixtures of them are not patient friendly due to the fact that missing medication is probable during drug treatment.

### Solution to Problem

It is an object of the present invention to provide stable solid oral pharmaceutical formulations comprising sevelamer or a pharmaceutically acceptable salt thereof and a fat soluble vitamin such as A, D, E or K or their analogues or mixtures thereof are in a single dosage form wherein the uniformity of content is fully achieved in the case where doses of two active pharmaceutical ingredients are significantly different from each other. This combination helps to treat vitamin A, D, E or K deficiency caused by hyperphosphatemia.

In the present invention, the content uniformity of single unit dosage pharmaceutical composition for combination of sevelamer and a vitamin A, D, E or K or their analogues is achieved with the use of surface active agents and/or wetteners in co-formulations. Since content uniformity of a vitamin A, D, E or K or their analogues within oral solid dosage form is improved, variability of in-vitro solubility within-tablet of active pharmaceutical ingredient is surpassed.

In the present invention, in order to achieve more stringent content uniformity in combination of sevelamer or a pharmaceutically acceptable salt thereof and a vitamin A, D, E or K or their analogues, new formulations are developed.

Uniformity of dosage units comprising sevelamer or a pharmaceutically acceptable salt thereof and a vitamin A, D, E or K or their analogues is determined at last according to US Pharmacopeia General Chapter <905>.

According to this invention, using sevelamer or a pharmaceutically acceptable salt thereof plus a vitamin A, D, E or K or their analogues combined pills provides benefits to patients and physicians. Such pharmaceutical agents are administered as part of the same dosage forms on the same administration schedule according to standard pharmaceutical practice.

Furthermore, sevelamer or a pharmaceutically acceptable salt thereof plus a vitamin A, D, E or K or their analogues in single dose combination will increase patient compliance. Combined pills improve physician and pharmacist efficiencies. As sevelamer or a pharmaceutically acceptable salt thereof and a vitamin A, D, E or K or their analogues are formulated together, the physician would write a single prescription. The pharmacist would dispense a single combination rather than multiple prescriptions.

### Description of embodiments

The invention relates to stable pharmaceutical formulations of sevelamer or a pharmaceutically acceptable salt thereof and a vitamin A, D, E or K or their analogues and a surface active agent or mixtures of surface active agents, in combination as a single dosage form which contributes to patient compliance. The single dosage formulation will provide convenience for the patient, where patients need multiple medications.

In this invention, the term of "vitamin D analogue" covers calcitriol (ergocalciferol (vitamin D₂)), cholecalciferol (vitamin D₃), doxercalciferol, 22-dihydroergocalciferol (vitamin D₄), sitocalciferol (vitamin D₅) or pharmaceutically acceptable salts thereof and combinations.

In this invention, the term of "vitamin A analogue" covers all forms of vitamin A, such as retinol (alcohol), retinal (aldehyde), retinyl acetate or retinyl palmitate (esters), and provitamin A carotenoids (b-carotene, a-carotene,) or combinations thereof.

In this invention, the term of "vitamin E analogue" covers existing naturally occurring vitamin E chemical forms (alpha-, beta-, gamma-, and delta-tocopherol and alpha-, beta-, gamma-, and delta-tocotrienol pr combinations thereof) that have varying levels of biological activity. Naturally sourced vitamin E is called d-alpha-tocopherol; the synthetically produced form is dl-alpha-tocopherol.

In this invention, the term of "vitamin E analogue" covers all existing forms of the natural vitamin K₁ and K₂, and synthetic forms K₃, K₄, K₅, K₆, K₇, K₈ , K₉, Vitamin K-S (II).

This invention covers all of fat soluble vitamins such as A, D, E or K or their analogues or mixtures thereof. Where the term of "A, D, E or K or their analogues or mixtures thereof" is used it covers all of the fat soluble vitamins.

In the present invention, it is aimed to administer sufficient amount of vitamin D analogue to treat vitamin D deficiency in one single dosage form together with sevelamer or a pharmaceutically acceptable salt thereof.

In need of vitamin D supplementation with sufficient vitamin D analogues, formulations avail to effectively and safely restore blood 25-hydroxyvitamin D levels to optimal levels (as equal to greater than 30 ng/ml) and maintain blood 25-hydroxy vitamin D levels at such optimal levels.

The desired dose of vitamin D analogue to be combined with sevelamer or a pharmaceutically acceptable salt thereof according to the invention is between 100 and 7000 IU per day, more preferably between 200 and 6000 IU and most preferably between 400 and 5500 IU.

Suitably tablet formulations of the invention may contain up to the maximum permitted daily dose or maximum permitted daily dosages can be administered as multiple dosage forms at different dosing times throughout day.

Monolithic tablet, bilayer tablet, inlay tablet, tablet in tablet, granules in sachet, active ingredient coated tablet formulations of sevelamer or a pharmaceutically acceptable salt thereof and a vitamin A, D, E or K or their analogues and a surface active agent or mixtures of surface active agents, are disclosed in the present invention to solve content uniformity problem.

A first embodiment of the present disclosure provides monolithic tablet of sevelamer or a pharmaceutically acceptable salt thereof and a vitamin A, D, E or K or their analogues and a surface active agent or mixtures of surface active agents combination where both low and high dose of different active pharmaceutical ingredients are homogenously distributed in the dosage form unit and devoid of separate/discrete layers in the formulation.

In the monolithic tablet, granules, particles or directly compressible powder of sevelamer or a pharmaceutically acceptable salt thereof are homogenously mixed with a vitamin A, D, E or K or their analogues thus co-formulation is obtained wherein formulation contains at least one surface active ingredient. A surface active agent is of necessity applied to improve solubility of a vitamin A, D, E or K or their analogues in the development of monolithic tablet for combination of sevelamer or a pharmaceutically acceptable salt thereof and a vitamin A, D, E or K or their analogues.

Single unit dose formulations, containing a surface active agent or mixtures of surface active agents ,may be prepared using methods as below without limiting scope of this invention;
- Sevelamer or pharmaceutically acceptable salt thereof and vitamin A, D, E or K or their analogues or mixtures thereof are separately granulated and then granulates are combined or,
- Sevelamer or pharmaceutically acceptable salt thereof or a vitamin A, D, E or K or their analogues or mixtures thereof are granulated and then granulate is combined with non granulated other one or,
- Sevelamer or pharmaceutically acceptable salt thereof and a vitamin A, D, E or K or their analogues or mixtures thereof are granulated together or,
- Sevelamer or pharmaceutically acceptable salt thereof or a vitamin A, D, E or K or their analogues or mixtures thereof are dissolved alone or with one or more excipients and then other one or with one or more excipients are coated with said solution
- Sevelamer or pharmaceutically acceptable salt thereof or a vitamin A, D, E or K or their analogues or mixtures thereof aredispersed alone or with one or more excipients and then other one or with one or more excipients are coated or adhered with said dispersion.

A second embodiment of the present disclosure provides bilayer tablet formulation of sevelamer or a pharmaceutically acceptable salt thereof and a vitamin A, D, E, K or their analogues combination containing a surface active agent or mixtures of surface active agents.

According to this invention, the bilayer tablet formulation for oral administration comprises a first layer which includes sevelamer or a pharmaceutically acceptable salt thereof and a second layer which includes vitamin A, D, E or K or their analogues alone or with sevelamer or a pharmaceutically acceptable salt thereof provided that the overall tablet contains sevelamer or a pharmaceutically acceptable salt thereof, a vitamin A, D, E or K or their analogues or mixtures thereof and particular amount of surface active agent. Sevelamer or a pharmaceutically acceptable salt thereof may be substantially or completely in different layers than vitamin A, D, E or K or in the same layer with a vitamin D analogue.

Single unit dosage form can be prepared as a bilayer tablet wherein a) a layer comprises a vitamin A, D, E or K or their analogues or mixtures thereof and a surface active agent or mixtures thereof and optionally additional excipient or excipients or b) a layer comprises a vitamin A, D, E or K or their analogues or mixtures thereof and a surface active agent or mixtures thereof and a portion of sevelamer or pharmaceutically acceptable salts thereof and optionally additional excipient or excipients or, c) a layer comprises a surface active agent or mixtures thereof and sevelamer or pharmaceutically acceptable salts thereof and optionally additional excipient or excipients, d)a layer comprises a portion of vitamin A, D, E or K or their analogues or mixtures thereof and a surface active agent or mixtures thereof and sevelamer or pharmaceutically acceptable salts thereof and optionally additional excipient or excipients .

In the present invention, bilayer tablet formulations are prepared by means of wet granulation where prepared intragranular and optional extragranular portions may coexist in both layers. The first layer may comprise intragranular and optionally extragranular portions where sevelamer or pharmaceutically acceptable salt thereof alone or in combination directly compressible sevelamer or pharmaceutically acceptable salt as in extragranular portion. As an alternative, the first layer may be a dry mixture formulation of sevelamer or pharmaceutically acceptable salt thereof .The second layer may comprise intragranular portion and optionally extragranular portion, where the intragranular portion comprising vitamin A, D, E or K or their analogues or mixtures thereof and at least a surface active agent ,alone or in combination with granulated or directly compressible sevelamer or pharmaceutically acceptable salt and optionally an extragranular portion comprising sevelamer or a pharmaceutically acceptable salt thereof.

The tablet formulations of the invention may also include one or more layers placed in between or outside of first and second layers.

A third embodiment of the present invention provides tablet-in-tablet formulation of sevelamer or a pharmaceutically acceptable salt thereof and a vitamin d analogue combination containing a surface active agent or mixtures of surface active agents. Tablet in tablet, also known as a "compression coated tablet" comprises an inner tablet that is covered by an outer coat that is compressed onto the inner tablet.

The main advantage of a tablet-in-tablet formulation compared to the bilayer formulation is that outer layer and the inner layer are in a fixed position and cannot be easily separated during storage and handling.

In tablet- in-tablet formulations, the inner tablets of a vitamin A, D, E or K or their analogues or mixtures thereof alone or in combination with sevelamer or a pharmaceutically acceptable salt thereof are compressed in a regular tablet press, optionally film coated. Tablet in tablet can be produced by using of conventional press-coating technology, such as Kilian™ tablet press.

Said tablet-in-tablets are produced on a specialized tablet press which is operated by a specialized pressing procedure, whereby inner tablets are fed into the tablet-in-tablet pressing machine with powder or granules of sevelamer or a pharmaceutically acceptable salt thereof alone or in combination with a vitamin d analogue .The press is operated again such that a inner tablet comprises from a vitamin A, D, E or K or their analogues or mixtures thereof and a surface active agent ,optionally in combination with sevelamer or a pharmaceutically acceptable salt thereof or moreover solely with sevelamer or a pharmaceutically acceptable salt particles. Preferably, in tablet in tablet formulations, sevelamer or a pharmaceutically acceptable salt thereof and a vitamin A, D, E or K or their analogues or mixtures thereof are co-formulated with a surface active agent both in inner and outer parts of the tablet-in-tablet.

An embedded tablet-in-tablet composition includes a core tablet wherein the core tablet preferably includes a vitamin A, D, E or K or their analogues or mixtures thereof and a surface active agent, alone or in combination with sevelamer or pharmaceutically acceptable salt thereof. The surrounding outer tablet applied there over comprises sevelamer or a pharmaceutically acceptable salt thereof.

Single unit dosage form can be prepared as a tablet-in-tablet wherein a) core tablet or outer tablet comprises a vitamin A, D, E or K or their analogues or mixtures thereof and a surface active agent or mixtures thereof and optionally additional excipient or excipients or b) core tablet or outer tablet comprises a vitamin A, D, E or K or their analogues or mixtures thereof and a surface active agent or mixtures thereof and a portion of sevelamer or pharmaceutically acceptable salts thereof and optionally additional excipient or excipients or, c) core tablet or outer tablet comprises a surface active agent or mixtures thereof and sevelamer or pharmaceutically acceptable salts thereof and optionally additional excipient or excipients,d) core tablet or outer tablet comprises a portion of vitamin A, D, E or K or their analogues or mixtures thereof and a surface active agent or mixtures thereof and sevelamer or pharmaceutically acceptable salts thereof and optionally additional excipient or excipients .

Another embodiment of the present provides inlay tablet formulation containing a surface active agent or mixtures of surface active agents , of sevelamer or a pharmaceutically acceptable salt thereof and a vitamin A, D, E or K or their analogues or mixtures thereof .

Inlay tablet is known to be a type of layered tablet in which instead the core tablet being completely surrounded by coating, top surface is completely exposed. While preparation, only the bottom of the die cavity is filled with a portion of sevelamer or a pharmaceutically acceptable salt thereof alone or in combination with a vitamin d analogue and core tablet containing vitamin A, D, E or K or their analogues or mixtures thereof and at least a surface active agent, optionally with sevelamer or a pharmaceutically acceptable salt thereof is placed upon it. Core tablet may contain solely sevelamer or a pharmaceutically acceptable salt thereof.

Single unit dosage form can be prepared as an inlay tablet wherein a) inner tablet or outer tablet comprises a vitamin A, D, E or K or their analogues or mixtures thereof and a surface active agent or mixtures thereof and optionally additional excipient or excipients or b) inner tablet or outer tablet comprises a vitamin A, D, E or K or their analogues or mixtures thereof and a surface active agent or mixtures thereof and a portion of sevelamer or pharmaceutically acceptable salts thereof and optionally additional excipient or excipients or, c) inner tablet or outer tablet comprises a surface active agent or mixtures thereof and sevelamer or pharmaceutically acceptable salts thereof and optionally additional excipient or excipients,d) inner tablet or outer tablet comprises a portion of vitamin A, D, E or K or their analogues or mixtures thereof and a surface active agent or mixtures thereof and sevelamer or pharmaceutically acceptable salts thereof and optionally additional excipient or excipients .

Vitamin A, D, E or K or their analogues or mixtures thereof are co formulated with a surface active agent to increase the solubility of lipophilic material.

A further embodiment of the present invention provides a pharmaceutical dosage form contains granules or powders of sevelamer or a pharmaceutically acceptable salt thereof in combination with granules or powders of a vitamin A, D, E or K or their analogues or mixtures thereof and a surface active agent. Pharmaceutical oral dosage form can be either granules or powder in a sachet. Granules can be readily dispersed or readily dissolved before administration.

In a further embodiment, a tablet comprising sevelamer or a pharmaceutically acceptable salt thereof is coated by applying a solution or dispersion containing vitamin A, D, E or K or their analogues or mixtures thereof and a surface active agent.The tablet comprising sevelamer or a pharmaceutically acceptable salt thereof can be further coated with other pharmaceutically acceptable film coating agents or ready-to-use formulations such as Opadry mixtures.

Also solution or dispersion of a vitamin A, D, E or K or their analogues or mixtures thereof can be prepared together with pharmaceutically acceptable film forming agents and then can be applied to core tablet of sevelamer or a pharmaceutically acceptable salt thereof.

By the present invention, it is now possible to provide sevelamer or a pharmaceutically acceptable salt thereof and a vitamin A, D, E or K or their analogues or mixtures thereof and a surface active agent in combination as a single unit pharmaceutical oral dosage form.

In aspect of this invention, the term of co-formulation is defined as co-processing, co-mixing, co-dissolving, co-dispersing, co-suspending, consecutively adding of a vitamin A, D, E or K or their analogues or mixtures thereof and a surface active agent without limiting the scope of the invention.

In another embodiment, a vitamin A, D, E or K or their analogues or mixtures thereof are co-formulated with at least a surface active agent and optionally an excipient or excipients in a suspension or solution and then other sevelamer or a pharmaceutically acceptable salt thereof alone or with one or more excipients are coated with said solution or suspension. Vice versa can also be carried out.

Another aspect of the present invention is further directed to manufacturing processes for preparing monolithic tablet, bilayer tablet, inlay tablet, tablet in tablet and granules in sachet formulations comprising sevelamer or a pharmaceutically acceptable salt thereof and a vitamin A, D, E or K or their analogues or mixtures thereof and a surface active agent or mixtures of surface active agents.

Said active pharmaceutical ingredients can be separately processed, co-processed, consecutively processed. The formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods. Direct compression and wet granulation in fluid bed granulator are preferred.

In the present invention, sevelamer or a pharmaceutically acceptable salt thereof can be granulated with a solution, suspension or dispersion containing a vitamin A, D, E or K or their analogues or mixtures thereof alone or with a surface active ingredient. Granulation can be achieved via spray granulation technique in which spraying and drying are performed simultaneously or using high or low shear granulation technique in which an additional drying step after granulation is necessary to remove solvent. Alternatively, one-pot processor, in which both granulation and drying are completed, for high or low shear granulation can be utilized. Microwave and/or vacuum drying techniques can also be used for drying of wet granules containing sevelamer or a pharmaceutically acceptable salt thereof and a vitamin A, D, E or K or their analogues or mixtures thereof.

Optionally, part of sevelamer or a pharmaceutically acceptable salt thereof can be dry granulated optionally together with additional excipient(s) using roller compaction and/or slug tablet press technique and remaining part of sevelamer or a pharmaceutically acceptable salt thereof can be granulated together with a vitamin A, D, E or K or their analogues or mixtures thereof.

Granulation solvent can be selected from pharmaceutically acceptable solvents or mixtures thereof. Water is preferred solvent but mixtures of water and other organic solvents can be used also. Granulation solvent can further contain additional functional excipients or cosolvents such as but not limited to binder(s), solubility enhancer(s), emulsifier(s), edible vegetable oil(s), absorption enhancer(s) or mixtures thereof.

Core tablet which contains sevelamer or a pharmaceutically acceptable salt thereof can be produced separately and further coated with a vitamin A, D, E or K or their analogues or mixtures thereof containing solution where a vitamin A, D, E or K or their analogues or mixtures thereof containing coating solution may optionally comprise surfactant and/or edible vegetable oils and/or pharmaceutically acceptable solvent. In mentioned solution, a vitamin A, D, E or K or their analogues or mixtures thereof can be completely or partially dissolved or suspended with aid of any functional agent for desired purpose.

Surfactants which can be used to completely or partially solubilise a vitamin A, D, E or K or their analogues or mixtures thereof are selected from non-ionic or ionic surfactants which are approved and safe for pharmaceutical and/or food supplements or combinations thereof. Examples of suitable surfactants, without limiting scope of the invention, are poloxamers, polyoxyethylene castor oil derivatives, polysorbates, polyoxyethylene stearates, sodium lauryl sulphate and sorbitan fatty acid esters, mixtures thereof and the like.

The Hydrophilic-lipophilic balance of a surfactant is a measure of the degree to which it is hydrophilic or lipophilic in a scale of 0 to 20. In the present invention, HLB values of surfactants to improve solubility of a vitamin A, D, E or K or their analogues or mixtures thereof vary from 0.5 to 40 or preferably from 5 to 20 or more preferably from 10 to 20.

Suspending agents also can be used in formulations. Suspending agent can be selected from the ones which are approved and safe for pharmaceutical and/or food supplements. Suspending agents can also have multi-functionality such as having both suspending and solubility enhancing characteristics at the same time.

Since a vitamin A, D, E or K or their analogues or mixtures thereof are insoluble or sparingly soluble in aqueous media; use of organic solvent is required most of the time. This solvent should be volatile, non toxic and removable to a level that is acceptable according to The International Committee on Harmonization (ICH) guidelines by drying.

Regulation has a strict limitation of the solvent residue in some granule products, such as in pharmaceuticals and food products. Published pharmacopoeias and ICH guidelines determine maximum allowable amounts of residual solvent in pharmaceutical products.

Suitable solvents which can be utilized for manufacturing pharmaceutical products of this invention can be selected from Class 1, 2 or 3 solvents mentioned in ICH Guideline IMPURITIES: GUIDELINE FOR RESIDUAL SOLVENTS Q3C (R4). Preferably selected solvents or mixtures thereof are from Class 2 or 3. More preferably, solvent or mixture of solvents is selected from Class 3.

Preferred pharmaceutical oral dosage form is tablet which can be optionally coated by methods well known in the art.

Examples to pharmaceutically acceptable film forming agents or ready-to-use mixtures, without limiting the scope of the invention, are hydroxypropylmethyl cellulose, polyvinyl alcohol. Film coating solution can also contain further functional excipients such as plasticizers, opacifiers and colouring agents.

In the present invention, sevelamer or a pharmaceutically acceptable salt thereof plus a vitamin A, D, E or K or their analogues or mixtures thereof in single dose combination can contain one or more excipients, such as diluents, binders, glidants, disintegrants, lubricants.

The amount of each type of additive employed, e.g. glidant, binder, disintegrant, filler or diluent and lubricant may vary within ranges conventional in the art.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Pharmaceutically acceptable additives other than those described above may be included in formulations of the present invention.

The compositions may include flavour enhancers, preservatives, antiadherants, flavourings, colouring agents, surfactants, solubilizers, wetting agents and other excipients of common use. Additives ingredients will vary according to the type of compositions being manufactured and can be included in the pharmaceutical composition in any amount.

The presented pharmaceutical formulations and manufacturing processes can be applied to other fat soluble vitamins.

In another aspect, newly developed formulations results proved improved content uniformity.

Although the USP (US Pharmacopeia) is not a regulatory body, their recommendations for testing protocols are readily adopted by pharmaceutical companies. In the case of content uniformity USP: 1.The actual % API in 10 tablets is tested and the acceptance value (AV) is calculated. The requirements for dosage uniformity are met if the acceptance value (AV) of the first 10 dosage units is less than or equal to L1.If the acceptance value is greater than L1, next 20 dosage units must be tested and the acceptance value must be calculated. The requirements are met if the final acceptance value of 30 dosage units is less than or equal to L1 and no individual content of the dosage unit is less than (1-L2*0.01)M or more than(1+L2*0.01)M as specified in the *Calculation ofAcceptance Value* under *Content Uniformity or under* Weight Variation.(L1 =15.0, L2=25.0 unless otherwise specified) . According to this invention, acceptance value of content uniformity is not greater than 10.0. Preferably, acceptance value is not greater than 7.0, more preferably acceptance value is not greater than 6 and most preferably acceptance value is not greater than 5.

The following regarding content uniformity clearly showed that the content uniformity is ensured results in Table 1.

**Table 1**

| | Content Uniformity Result of vitamin D3 without surfactant | Content Uniformity Result of vitamin D3 formulated with %1 Sodium Lauryl Sulfate(%) |
|---|---|---|
| Sample -1 | 96,1 | 97,4 |
| Sample -2 | 92,4 | 103,4 |
| Sample -3 | 90,9 | 101,6 |
| Sample -4 | 106,8 | 98,7 |
| Sample -5 | 88,3 | 101,8 |
| Sample -6 | 103,9 | 99,8 |
| Sample -7 | 94,8 | 103,6 |
| Sample -8 | 97,2 | 97,2 |
| Sample -9 | 93,7 | 102,7 |
| Sample -10 | 87,9 | 99,5 |
| Mean | 95,2 | 100,6 |
| SD | 6,2 | 2,4 |
| RSD% | 6,5 | 2,4 |
| AV | 18,2 | 5,8 |

A suitable dissolution method is used to reach dissolution profiles for vitamin D analogue. In this invention USP Type-2 apparatus is preferred and used. According to the method which was developed during drug development process, tablets prepared in accordance with the present invention should exhibit the following dissolution profile when tested in a USP Type 2 apparatus, at 75 rpm, and 37°C and in 0.3% SDS medium.

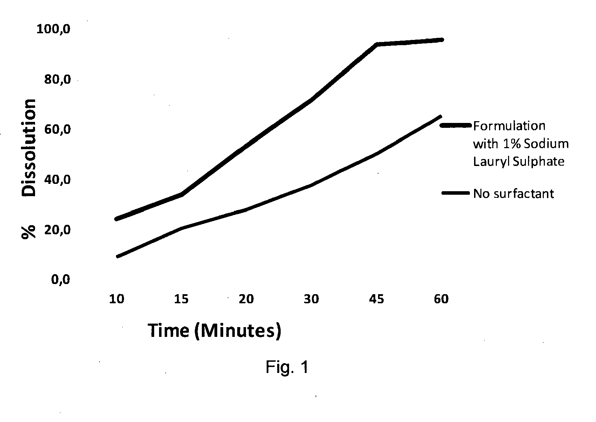

Figure 1, Table 2 and 3 clearly summarize below the dissolution profiles obtained with or without addition of %1 sodium lauryl sulphate in the formulation. Use of appropriate amount of surfactant clearly proves in solubility improvements of vitamin D analogue.

**Table 2**

| **Time(min)** | **10** | **15** | **20** | **30** | **45** | **60** |
|---|---|---|---|---|---|---|
| **% Dissolved vitamin D3 no surfactant** | | | | | | |
| Vessel 1 | 8,5 | 21,0 | 28,4 | 36,9 | 49,8 | 63,5 |
| Vessel 2 | 12,3 | 24,2 | 31,6 | 42,1 | 55,0 | 70,8 |
| Vessel 3 | 5,9 | 15,5 | 23,0 | 33,7 | 46,1 | 62,3 |
| Average | 8,9 | 20,2 | 27,7 | 37,6 | 50,3 | 65,5 |
| Min. | 5,9 | 15,5 | 23,0 | 33,7 | 46,1 | 62,3 |
| Max | 12,3 | 24,2 | 31,6 | 42,1 | 55,0 | 70,8 |
| SD | 3,2 | 4,4 | 4,3 | 4,2 | 4,5 | 4,6 |
| RSD | 36,2 | 21,7 | 15,7 | 11,3 | 8,9 | 7,0 |

**Table 3**

| | | | | | | |
|---|---|---|---|---|---|---|
| **Time(min)** | **10** | **15** | **20** | **30** | **45** | **60** |

| **Formulation with 1 % Sodium Lauryl Sulphate** | | | | | | |
|---|---|---|---|---|---|---|
| Vessel 1 | 19,5 | 28,0 | 49,3 | 69,8 | 92,5 | 95,1 |
| Vessel 2 | 27,0 | 39,1 | 58,4 | 74,5 | 95,7 | 97,4 |
| Vessel 3 | 25,1 | 34,9 | 52,5 | 70,6 | 93,0 | 94,5 |
| Average | 23,9 | 34,0 | 53,4 | 71,6 | 93,7 | 95,7 |
| Min. | 19,5 | 28,0 | 49,3 | 69,8 | 92,5 | 94,5 |
| Max. | 27,0 | 39,1 | 58,4 | 74,5 | 95,7 | 97,4 |
| SD | 3,9 | 5,6 | 4,6 | 2,5 | 1,7 | 1,5 |
| RSD | 16,3 | 16,5 | 8,6 | 3,5 | 1,8 | 1,6 |

The following examples are given for the purpose of illustration of the present invention and should not limit the scope of the invention.

### Example 1: Monolithic tablet Unit Formulation

**Table 4**

| **Ingredients** | **mg/tablet** | **%(w/w)** |
|---|---|---|
| Sevelamer HCl | 800.00 | 80.000 |
| Vitamin D3 | 0.01 | 0.001 |
| HPMC E5 | 18.00 | 1.800 |
| Diacetylated Monoglyceride | 8.50 | 0.850 |
| Colloidal Silicon Dioxide | 107.50 | 10.750 |
| Stearic Acid | 13.50 | 1.350 |
| Cremophor ELP | 8.49 | 0.849 |
| Olive Oil | 44.00 | 4.400 |
| **Total Core Tablet Weight** | **1,000.00** | **100.000** |

### Example 2: Monolithic tablet Manufacturing Method of Example 1

Pharmaceutical formulation may be prepared using methods as below without limiting scope of this invention;
1. Vitamin D3 is dissolved in Olive Oil together with Cremophor ELP and Diacetylated Monoglyceride.
2. HPMC E5 is dissolved in sufficient quantity of water.
3. Vitamin D₃ solution is added to HPMC E5 solution and mixed until homogeneous mixture is achieved.
4. Sevelamer HCl is granulated with dispersion prepared in step 3 using Glatt GPCG 3 fluid bed granulator.
5. Granules are further dried until moisture content is less than 1 % (w/w).
6. Colloidal Silicon Dioxide is mixed with some part of dry granules, sieved through suitable sized sieve.
7. Remaining dry granules are mixed with Colloidal Silicon Dioxide mixture prepared in step (6).
8. Stearic Acid is added and mixed.
9. Core tablets are compressed with suitable punches having average target weight of 1,000 mg/tablet.

### Example 3: Bilayer tablet Unit Formulation

**Table 5**

| **Ingredients** | **mg/tablet** | **% (w/w)*** |
|---|---|---|
| Sevelamer Carbonate | 800.00 | 80.000 |
| Povidone (K-30) | 23.50 | 2.350 |
| Colloidal Silicon Dioxide | 117.00 | 11.700 |
| Stearic Acid | 9.50 | 0.950 |
| **Total First Layer Weight** | **950.00** | |
| Vitamin D3 | 0.01 | 0.001 |
| Sodium Lauryl Sulphate | 0.50 | 0.05 |
| Microcrystalline Cellulose (Avicel PH 101) | 48.99 | 4.899 |
| Glyceryl Behenate | 0.50 | 0.050 |
| **Total Second Layer Weight** | **50.00** | |
| **Total Core Tablet Weight** | **1,000.00** | |

| | | |
|---|---|---|
| * % values are calculated according to final core tablet weight. | | |

### Example 4:Bilayer Tablet Manufacturing Method of Example 3

Pharmaceutical formulation may be prepared using methods as below without limiting scope of this invention;
1. Sevelamer Carbonate, Povidone K-30 and half of Colloidal Silicon Dioxide are mixed.
2. Powder mixture in step (1) is dry granulated using Powtec roller compactor.
3. Compacted granules are sieved through suitable sized sieve and mixed again.
4. Remaining part of Colloidal Silicon Dioxide and part of granules from step (3) are mixed and sieved through suitable sized sieve.
5. Colloidal Silicon Dioxide mixture is added to remaining dry granules and mixed.
6. Stearic Acid is added and mixed to powder mixture in step (5).
7. Vitamin D₃ is dissolved in water together with Sodium Lauryl Sulphate.
8. Avicel PH 101 is granulated using Vector GMX Micro HSM with solution prepared in step (7).
9. Wet granules are dried in an oven at ambient temperature which has vacuuming function until moisture content is less than 4% (w/w).
10. Dry granules in step (9) is sieved through suitable sized sieve and mixed.
11. Glyceryl Behenate is added to dry granules in step 10 and mixed.
12. Double layered tablets are compressed from powder mixtures in step (5) and (11) using Fette 102i tablet press machine.

### Example 5:Inlay Tablet Unit Formulation

**Table 6**

| **Ingredients** | **mg/tablet** | **% (w/w)*** |
|---|---|---|
| Sevelamer Carbonate | 800.00 | 80.000 |
| Povidone (K-30) | 25 | 2.500 |
| Colloidal Silicon Dioxide | 113 | 11.300 |
| Stearic Acid | 12 | 1.200 |
| **Total Outer Tablet Weight** | **950.00** | |
| Vitamin D3 | 0.01 | 0.001 |
| Sodium Lauryl Sulphate | 0.50 | 0.050 |
| Microcrystalline Cellulose (Avicel PH 101) | 48.99 | 4.899 |
| Glyceryl Behenate | 0.50. | 0.050 |
| **Total Inlay Tablet Weight** | **50.00** | |
| **Total Core Tablet Weight** | **1,000.00** | |

### Example 6:Inlay Tablet Manufacturing Method of Example 5

Pharmaceutical formulation may be prepared using methods as below without limiting scope of this invention;
1. Vitamin D₃ is dissolved in water together with Sodium Lauryl Sulphate.
2. Avicel PH 101 is granulated using Vector GMX Micro HSM with solution prepared in step (1).
3. Wet granules are dried in an oven at ambient temperature which has vacuuming function until moisture content is less than 4% (w/w).
4. Dry granules in step (3) is sieved through suitable sized sieve and mixed.
5. Glyceryl Behenate is added to dry granules in step (4) and mixed.
6. Inlay tablets are compressed from powder mixtures in step (5) using Fette 102i tablet press machine.
7. Sevelamer Carbonate, Povidone K-30 and half of Colloidal Silicon Dioxide are mixed.
8. Powder mixture in step (7) is dry granulated using Powtec roller compactor.
9. Compacted granules are sieved through suitable sized sieve and mixed again.
10. Remaining part of Colloidal Silicon Dioxide and part of granules from step (9) are mixed and sieved through suitable sized sieve.
11. Colloidal Silicon Dioxide mixture is added to remaining dry granules and mixed.
12. Stearic Acid is added and mixed to powder mixture in step (11).
13. Inlay tablets are compressed from powder mixtures in step (12) and tablets prepared in step (6) using Fette 102i tablet press machine.

### Example 7: Tablet in tablet Unit Formulation

**Table 7**

| **Ingredients** | **mg/tablet** | **% (w/w)*** |
|---|---|---|
| Sevelamer Carbonate | 450.00 | 45.0 |
| Povidone (K-30) | 22.50 | 2.25 |
| Micro crystalline cellulose | 32.00 | 3.20 |
| Colloidal Silicon Dioxide | 65.00 | 6.50 |
| Stearic Acid | 10.50 | 1.05 |
| **TotalOuterTablet Weight** | **580.00** | |
| Vitamin D3 | 0.01 | 0.001 |
| Sevelamer Carbonate | 350.00 | 35.00 |
| Olive Oil | 19.25 | 1.92 |
| Cremophor ELP | 3.80 | 0.38 |
| Diacetylated Monoglyceride | 3.70 | 0.37 |
| HPMC E5 | 8.00 | 0.80 |
| Colloidal Silicon Dioxide | 29.29 | 2.929 |
| Stearic Acid | 6.00 | 0.60 |
| **Total Inner Layer Weight** | **420.00** | |
| **Total CoreTablet Weight** | **1,000.00** | |

### Example 8:Tablet in tablet Manufacturing Method of Example 7

Pharmaceutical formulation may be prepared using methods as below without limiting scope of this invention;
1. Vitamin D3 is dissolved in Olive Oil together with Cremophor ELP and Diacetylated Monoglyceride.
2. HPMC E5 is dissolved in sufficient quantity of water.
3. Vitamin D₃ solution is added to HPMC E5 solution and mixed until homogeneous mixture is achieved.
4. Sevelamer HCl is granulated with dispersion prepared in step 3 using Glatt GPCG 3 fluid bed granulator.
5. Granules are further dried until moisture content is less than 1 % (w/w).
6. Colloidal Silicon Dioxide is mixed with some part of dry granules, sieved through suitable sized sieve.
7. Remaining dry granules are mixed with Colloidal Silicon Dioxide mixture prepared in step (6).
8. Stearic Acid is added and mixed.
9. Inner tablets are compressed with suitable punches having average target weight of 420 mg/tablet.
10. Sevelamer Carbonate, Povidone K-30 and half of Colloidal Silicon Dioxide of outer tablet are mixed.
11. Powder mixture in step (10) is dry granulated using Powtec roller compactor.
12. Compacted granules are sieved through suitable sized sieve and mixed again.
13. Remaining half part of Colloidal Silicon Dioxide of outer tablet, part of granules from step (12) and microcrystalline cellulose are mixed and sieved through suitable sized sieve.
14. Stearic Acid is added and mixed to powder mixture in step (13).
15. Total core tablets are compressed from powder mixtures in step (14) using Fette 102i tablet press machine.

### Example 9: Granules in Sachet Unit Formulation

**Table 8**

| **Ingredients** | **mg/sachet** | **%(w/w)** |
|---|---|---|
| Sevelamer Carbonate | 2,400.00 | 80.000 |
| Vitamin D3 | 0.04 | 0.001 |
| Colloidal Silicon Dioxide | 317.00 | 10.568 |
| Sucralose | 45.00 | 1.500 |
| Cherry Flavour | 55.00 | 1.833 |
| Sodium Chloride | 25.00 | 0.833 |
| Xanthan Gum | 70.00 | 2.333 |
| Cremophor ELP | 17.96 | 0.599 |
| Sunflower Oil | 70.00 | 2.333 |
| **Total Weight of Powder in Sachet** | **3,000.00** | **100.00** |

### Example 10: Granules in Sachet Manufacturing Method of Example 9

Pharmaceutical formulation may be prepared using methods as below without limiting scope of this invention;
1. Xanthan Gum, Sucralose, one third of Colloidal Silicon Dioxide and Cherry Flavour are mixed.
2. Powder mixture in step 1 is sieved through suitable sized sieve and mixed.
3. Half of Sevelamer Carbonate, powder mixture in step (2) and half of Sevelamer Carbonate are added to Collette UltimaGral 25 and mixed.
4. Vitamin D3 and Cremophor ELP are dissolved in Sunflower Oil.
5. Sodium Chloride is dissolved in water.
6. Vitamin D3 solution is added to Sodium Chloride solution and mixed.
7. Powder mixture in step 3 is granulated with dispersion prepared in step (6)
8. Wet granules are dried in Collette UltimaGral 25 using both microwave and vacuuming functions until moisture content is less than 1%.
9. Dried granules are sieved through suitable sized sieve and mixed.
10. Remaining Colloidal Silicon Dioxide and some part of dried granules are mixed and sieved through suitable sized sieve.
11. Powder mixture in step (10) is mixed with remaining dried granules and mixed.
12. Final powder mixture is filled into suitable packaging materials using sachet filling machine at average target powder weight of 3,000 mg/sachet.

## Claims

1. A single unit dosage pharmaceutical composition comprising sevelamer or pharmaceutically acceptable salts thereof and a fat soluble vitamin or mixtures of vitamins **characterized in that** said composition comprises at least sevelamer or pharmaceutically acceptable salts thereof and a surface active agent or mixtures of surface active agents and a fat soluble vitamin or mixtures of fat soluble vitamins.

2. A single unit dosage pharmaceutical composition according to claim 1 wherein Fat soluble vitamin is selected from the group consisting of A, D, E or K or analogues or mixtures thereof.

3. A single unit dosage pharmaceutical composition according to claims 1 to 2 wherein surface active agent or mixtures of surface active agents are co-formulated with sevelamer or pharmaceutically acceptable salts thereof and a vitamin or mixtures of vitamins selected from the group consisting of A, D, E or K.

4. A single unit dosage pharmaceutical composition according to claims 1 to 2 wherein, surface active agent or mixtures of surface active agents are formulated in single unit dosage form with a) a vitamin or mixtures of vitamins selected from the group consisting of A, D, E or K or analogues thereof or, b) sevelamer or pharmaceutically acceptable salts thereof and a vitamin or mixtures of vitamins selected from the group consisting of A, D, E or K or, c)sevelamer or pharmaceutically acceptable salts thereof

5. A single unit dosage pharmaceutical composition according to claims 1 to 2 wherein, single unit dosage form, containing a surface active agent or mixtures of surface active agents, is prepared as a monolithic tablet wherein a preparation method by followings or mixtures of them are used: a) Sevelamer or pharmaceutically acceptable salt thereof and vitamin A, D, E or K or their analogues or mixtures thereof are separately granulated and then granulates are combined or, b) Sevelamer or pharmaceutically acceptable salt thereof or a vitamin A, D, E or K or their analogues or mixtures thereof are granulated and then granulate is combined with non granulated other one or, c) Sevelamer or pharmaceutically acceptable salt thereof and a vitamin A, D, E or K or their analogues or mixtures thereof are granulated together or, d) Sevelamer or pharmaceutically acceptable salt thereof or a vitamin A, D, E or K or their analogues or mixtures thereof are dissolved alone or with one or more excipients and then other one or with one or more excipients are coated with said solution, e) Sevelamer or pharmaceutically acceptable salt thereof or a vitamin A, D, E or K or their analogues or mixtures thereof are dispersed alone or with one or more excipients and then other one or with one or more excipients are coated or adhered with said dispersion.

6. A single unit dosage pharmaceutical composition according to claims 1 to 2 in which single unit dosage form is prepared as a bilayer tablet wherein a) a layer comprises a vitamin A, D, E or K or their analogues or mixtures thereof and a surface active agent or mixtures thereof and optionally additional excipient or excipients or b) a layer comprises a vitamin A, D, E or K or their analogues or mixtures thereof and a surface active agent or mixtures thereof and a portion of sevelamer or pharmaceutically acceptable salts thereof and optionally additional excipient or excipients or, c) a layer comprises a surface active agent or mixtures thereof and sevelamer or pharmaceutically acceptable salts thereof and optionally additional excipient or excipients, d)a layer comprises a portion of vitamin A, D, E or K or their analogues or mixtures thereof and a surface active agent or mixtures thereof and sevelamer or pharmaceutically acceptable salts thereof and optionally additional excipient or excipients .

7. A single unit dosage pharmaceutical composition according to claims 1 to 2 in which, single unit dosage form is prepared as a tablet-in-tablet wherein a) core tablet or outer tablet comprises a vitamin A, D, E or K or their analogues or mixtures thereof and a surface active agent or mixtures thereof and optionally additional excipient or excipients or b) core tablet or outer tablet comprises a vitamin A, D, E or K or their analogues or mixtures thereof and a surface active agent or mixtures thereof and a portion of sevelamer or pharmaceutically acceptable salts thereof and optionally additional excipient or excipients or, c) core tablet or outer tablet comprises a surface active agent or
mixtures thereof and sevelamer or pharmaceutically acceptable salts thereof and optionally additional excipient or excipients,d) core tablet or outer tablet comprises a portion of vitamin A, D, E or K or their analogues or mixtures thereof and a surface active agent or mixtures thereof and sevelamer or pharmaceutically acceptable salts thereof and optionally additional excipient or excipients .

8. A single unit dosage pharmaceutical composition according to claims 1 to 2 in which single unit dosage form is prepared as an inlay tablet wherein a) inner tablet or outer tablet comprises a vitamin A, D, E or K or their analogues or mixtures thereof and a surface active agent or mixtures thereof and optionally additional excipient or excipients or b) inner tablet or outer tablet comprises a vitamin A, D, E or K or their analogues or mixtures thereof and a surface active agent or mixtures thereof and a portion of sevelamer or pharmaceutically acceptable salts thereof and optionally additional excipient or excipients or, c) inner tablet or outer tablet comprises a surface active agent or mixtures thereof and sevelamer or pharmaceutically acceptable salts thereof and optionally additional excipient or excipients,d) inner tablet or outer tablet comprises a portion of vitamin A, D, E or K or their analogues or mixtures thereof and a surface active agent or mixtures thereof and sevelamer or pharmaceutically acceptable salts thereof and optionally additional excipient or excipients .

9. A single unit dosage pharmaceutical composition according to claims 1 to 2 in which single unit dosage form is prepared as granules or powders in sachet wherein sevelamer or a pharmaceutically acceptable salt thereof and a vitamin selected from the group consisting of A, D, E or K or their analogues or mixtures thereof are co-formulated with a surface active agent

10. A single unit dosage pharmaceutical composition according to claims_1 to 9,
used surface active agent or mixture of surface active agents, exhibit a hydrophilic-lipophilic balance value from 0.5 to 40, preferably from 5 to 20 and more preferably from 10 to 20.

11. A single unit dosage pharmaceutical composition according to preceding
claims, in single unit dosage pharmaceutical compositions, acceptance value of vitamin A, D, E or K or their analogues or mixtures thereof is not greater than 10.0, preferably acceptance value is not greater than 7.0, more preferably acceptance value is not greater than 6 and most preferably acceptance value is not greater than 5, in terms of content uniformity.

## Patentansprüche

1. Eine einzelne Einheitsdosierung pharmazeutische Zusammensetzung umfassend Sevelamer oder pharmazeutisch annehmbare Salze davon und eine fettlösliche Vitamin oder Mischungen davon **dadurch gekennzeichnet, dass** die Zusammensetzung umfasst mindestens Sevelamer oder pharmazeutisch annehmbare Salze davon und ein en oberflächenaktiver Stoff oder Mischungen von oberflächenaktive Stoffe und ein fettlösliches Vitamin oder Mischungen der fettlöslichen Vitamine.

2. Eine einzelne Einheitsdosierung pharmazeutische Zusammensetzung nach Anspruch 1, wobei fettlösliches Vitamin ist ausgewählt aus der Gruppe bestehend aus A, D, E oder K oder Analoga oder Mischungen davon.

3. Eine einzelne Einheitsdosierung pharmazeutische Zusammensetzung nach Anspruch 1 bis 2 wobei oberflächenaktiver Stoff oder Mischungen von oberflächenaktiven Stoffe mit Sevelamer oder pharmazeutisch annehmbare Salze davon und ein Vitamin oder Mischungen von Vitaminen ausgewählt aus der Gruppe bestehend aus A, D, E zusammen formuliert.

4. Eine einzelne Einheitsdosierung pharmazeutische Zusammensetzung nach Ansprüchen 1 bis 2, wobei aktiver oberflächenaktiver Stoff oder Mischungen von aktiven oberflächenaktiven Stoffe in der einzelnen Einheitsdosierungsform mit a) ein Vitamin oder Mischungen von Vitaminen ausgewählt aus der Gruppe bestehend aus A, D, E oder K oder Analoga davon oder, b) sevelamer oder pharmazeutisch annehmbare Salze davon und ein Vitamin oder Mischungen von Vitaminen ausgewählt aus der Gruppe bestehend aus A, D, E oder K oder, c) sevelamer oder pharmazeutisch annehmbare Salzen davon.

5. Eine einzelne Einheitsdosierung pharmazeutische Zusammensetzung nach Ansprüche 1 bis 2, wobei einzelne Einheitsdosierungsform enthaltend einen oberflächenaktiven Stoff oder Mischungen von oberflächenaktiven Stoffe, wird als eine monolithische Tablette zubereitet, wobei eine Zubereitungsmethode durch Folgende oder Mischungen davon verwendet wird:
a) Sevelamer oder pharmazeutisch annehmbares Salz davon und Vitamin A, D, E oder K oder ihre Analoga oder Mischungen davon werden getrennt granuliert, und danach werden die Granulate zusammengelegt oder,
b) Sevelamer oder pharmazeutisch annehmbares Salz davon oder ein Vitamin A, D, E oder K oder ihre Analoga oder Mischungen davon werden granuliert und dann werden die Granulate mit nichtgranuliert andere zusammengelegt oder, c) Sevelamer oder pharmazeutisch annehmbares Salz davon und ein Vitamin A, D, E oder K oder ihre Analoga oder Mischungen davon werden zusammen granuliert oder, d) Sevelamer oder pharmazeutisch annehmbares Salz davon oder ein Vitamin A, D, E oder K oder ihre Analoga oder Mischungen davon werden allein oder mit einem oder mehr Hilfstoffe aufgelöst, und dann anderer oder mit einem oder mehr Hilfstoffe werden mit der gesagten Lösung beschichtet, e) Sevelamer oder pharmazeutisch annehmbares Salz davon oder ein Vitamin A, D, E oder K oder Analoga oder Mischungen davon werden allein oder mit einem oder mehreren Hilfsstoffe dispergiert und dann andere oder mit einem oder mehreren Hilfsstoffe werden beschichtet oder überzogen mit gesagte Dispersion.

6. Eine einzelne Einheitsdosierung pharmazeutische Zusammensetzung nach Ansprüche 1 bis 2 in welcher einzelne Einheitsdosierungsform ist als eine Zweischichttablette vorbereitet wobei a) eine Schicht umfasst ein Vitamin A, D, E oder K oder ihre Analoga oder Mischungen davon und einen oberflächenaktiven Stoff oder Mischungen davon und wahlweise zusätzlichen Hilfsstoff oder Hilfsstoffe oder b) eine Schicht umfasst ein Vitamin A, D, E oder K oder ihre Analoga oder Mischungen davon und einen oberflächenaktiven Stoff oder Mischungen davon und ein Teilen von sevelamer oder pharmazeutisch annehmbare Salze davon und wahlweise zusätzlichen Hilfsstoff oder Hilfsstoffe oder, c) eine Schicht umfasst einen oberflächenaktiven Stoff oder Mischungen davon und sevelamer oder pharmazeutisch annehmbare Salze davon und wahlweise zusätzlichen Hilfsstoff oder die Hilfsstoffe, d) eine Schicht umfasst einen Teil von Vitamin A, D, E oder K oder Analoga oder Mischungen davon und einen oberflächenaktiven Stoff oder Mischungen davon und Sevelamer oder pharmazeutisch annehmbare Salze davon und wahlweise zusätzlichen Hilfsstoff oder die Hilfsstoffe.

7. Eine einzelne Einheitsdosierung pharmazeutische Zusammensetzung nach Ansprüche 1 bis 2 in welcher einzelne Einheitsdosierungsform ist als Tablette in einer Tablette zubereitet wobei a) Kerntablette oder Außentablette umfasst ein Vitamin A, D, E oder K oder ihre Analoga oder Mischungen davon und einen oberflächenaktiven Stoff oder Mischungen davon und wahlweise zusätzlichen Hilfsstoff oder Hilfsstoffe oder b) Kerntablette oder Außentablette umfasst ein Vitamin A, D, E oder K oder ihre Analoga oder Mischungen davon und einen oberflächenaktiven Stoff oder Mischungen davon und einen Teil von Sevelamer oder pharmazeutisch annehmbare Salze davon und wahlweise zusätzlichen Hilfsstoff oder Hilfsstoffe oder, c) Kerntablette oder Außentablette umfasst einen oberflächenaktiven Stoff oder Mischungen davon und Sevelamer oder pharmazeutisch annehmbare Salze davon und wahlweise zusätzlichen Hilfsstoff oder Hilfsstoffe, d) Kerntablette oder Außentablette umfasst einen Teil von Vitamin A, D, E oder K oder Analoga oder Mischungen davon und einen oberflächenaktiven Stoff oder Mischungen davon und Sevelamer oder pharmazeutisch annehmbare Salze davon und wahlweise zusätzlichen Hilfsstoff oder Hilfsstoffe.

8. Eine einzelne Einheitsdosierung pharmazeutische Zusammensetzung nach Ansprüche 1 bis 2 in welcher einzelne Einheitsdosierungsform als eine Tablette mit Einlage zubereitet wird , worin a) innere Tablette oder äußere
Tablette umfasst einen Vitamin A, D, E oder K oder ihre Analoga oder Mischungen davon und einen oberflächenaktiven Stoff oder Mischungen davon und wahlweise zusätzlichen Hilfsstoff oder Hilfsstoffe oder b) innere Tablette oder äußere Tablette umfasst ein Vitamin A, D, E oder K oder ihre Analoga oder Mischungen davon und einen oberflächenaktiven Stoff oder Mischungen davon und einen Teil von Sevelamer oder pharmazeutisch annehmbare Salze davon und wahlweise zusätzlichen Hilfsstoff oder Hilfsstoffe oder c) innere Tablette oder äußere Tablette umfasst einen oberflächenaktiven Stoff oder Mischungen davon und Sevelamer oder pharmazeutisch annehmbare Salze davon und wahlweise zusätzlichen Hilfsstoff oder Hilfsstoffe, d) die innere Tablette oder äußere Tablette umfasst einen Teil von Vitamin A, D, E oder K oder ihre Analoga oder Mischungen davon und einen oberflächenaktiven Stoff oder Mischungen davon und Sevelamer oder pharmazeutisch annehmbare Salze davon und wahlweise zusätzlichen Hilfsstoff oder Hilfsstoffe.

9. Eine einzelne Einheitsdosierung pharmazeutische Zusammensetzung nach Ansprüche 1 bis 2, in welcher einzelne Einheitsdosierungsform wird als Granulate oder Pulver im Beutel zubereitet, worin sevelamer oder ein pharmazeutisch annehmbares Salz davon und ein Vitamin ausgewählt aus der Gruppe bestehend aus A, D, E oder K oder ihren Analoga oder Mischungen davon mit einem oberflächenaktiven Stoff zusammenformuliert werden.

10. Eine einzelne Einheitsdosierung pharmazeutische Zusammensetzung nach Ansprüche 1 zu 9, verwendeter oberflächenaktiver Stoff oder Mischung von oberflächenaktiven Stoffe, stellt eine hydrophil -lipophil Balance von 0.5 bis 40, vorzugsweise von 5 bis 20 und besonders bevorzugt von 10 bis 20 aus.

11. Eine einzelne Einheitsdosierung pharmazeutische Zusammensetzung nach vorhergehenden Ansprüche, in einzelner Einheitsdosierung pharmazeutische Zusammensetzungen, Annahmewert von Vitamin A, D, E oder K oder ihrer Analoga oder Mischungen davon ist nicht größer als 10.0, vorzugsweise ist Annahmewert nicht größer als 7.0, besonders bevorzugt ist Annahmewert nicht größer als 6 und am bevorzugtesten ist Annahmewert nicht größer als 5, in Bezug auf Gehalt-Gleichmäßigkeit.

## Revendications

1. Une seule dose unitaire de composition pharmaceutique comprenant sevelamer ou les sels pharmaceutiquement acceptables de colle-ci et une vitamine liposoluble ou les mélanges de vitamines **caractérisés en ce que** la dite composition comprend toujours sevelamer ou les sels pharmaceutiquement acceptables de celle-ci et un agent tensio-actif ou les mélanges des agents tensio-actifs et une vitamine liposoluble ou les mélanges de vitamines liposoubles.

2. Une seule dose unitaire de composition pharmaceutique selon la revendication 1, dans laquelle la vitamine liposoluble est choisie dans le groupe constitué de A, D, E ou K ou les analogues ou les élanges de celles-ci.

3. Une seule dose unitaire de composition pharmaceutique selon les revendications 1 à 2, dans laquelle l'agent tensio-actif ou des mélanges d'agents tensio-actifs sont formulés conjointement par sevelamer ou les sels pharmaceutiquement acceptables de celles-ci et une vitamine ou les mélanges de vitamines choisis dans le groupe constitué de A, D, E ou K.

4. Une seule dose unitaire de composition pharmaceutique selon les revendications 1 à 2, dans la quelle l'agent tensio-actif ou des mélanges d'agents tensio-actifs sont formulés en forme d'une seule dose unitaire avec a) une vitamine ou les mélanges de vitamines choisis dans le groupe constitué de A, D, E ou K ou ses analogues b) sevelamer ou les pharmaceutiquement acceptables de celle-ci et une vitamine liposoluble ou les mélanges de vitamines choisis dans le groupe constitué de A, D, E ou K ou, c) sevelamer ou les sels pharmaceutiquement acceptables de celle-ci.

5. Une seule dose unitaire de composition pharmaceutique selon les revendications 1 à 2, dans la quelle la seule dose unitaire contenant un agent tensio-actif ou des mélanges d'agents tensio-actifs ost préparée comme un comprimé monolithique dans le quel une méthode de préparation suivant ou les mélanges de celles-ci sont utilisés : a) sevelamer ou les sels pharmaceutiquement acceptables de celles-ci et vitamines A, D, E ou K ou les analogues ou les mélanges de celles-ci sont séparément granulés puis les granules sont combinés ou b) sevelamer ou les pharmaceutiquement acceptables de celle-ci ou une vitamine A, D, E ou K ou les analoguos ou les mélanges de celles-ci sont granulés puis les granules sont combinés avec les autres non granulés ou, c) sevelamer ou les sels pharmaceutiquement acceptables de celle-ci ou une vitamine A, D, E ou K ou les analogues ou les mélanges de celles-ci sont granulés ensemble ou, d) sevelamer ou les sels pharmaceutiquement acceptables de celle-ci ou une vitamine A, D, E ou K ou les analogues ou les mélanges de celles-ci sont dissous seuls ou avec un ou plus d'excipients et puis un autre ou un ou plus d'excîpients sont revêtus avec la dite solution, e) sevelamer ou les sels pharmaceutiquement acceptables de celle-ci ou une vitamine A, D, E ou K ou les analogues ou les mélanges de celles-ci sont disséminés seuls ou avec un ou plus d'excipients et puis un autre ou un ou plus d'excipients sont revêtus ou adhérés avec la dispersion.

6. Une seule dose unitaire de composition pharmaceutique selon les revendications 1 à 2 dans laquelle la seule dose unitaire est préparée en bicouche comprimé où a) une couche comprend une vitamine A, D, E ou K ou les analogues ou les mélanges de celles-ci et un agent tensio-actif ou des mélanges d'agents tensio-actifs et facultativement oxcipient ou excipients supplémentaires ou b) une couche comprend une vitamine A, D, E ou K ou les analogues ou les mélanges de celles-ci et une partie de sevelamer ou les sels pharmaceutiquement acceptables de celle-ci et facultativement excipient ou excipients supplémentaires ou, c) une couche comprend un agent tonsio-actif ou des mélanges d'agents tensio-actifs et sevelamer ou les sels pharmaceutiquement acceptables de celle-ci et facultativement excipient ou excipients supplémentaires, d) une couche comprend une partie de vitamine A, D, E ou K ou les analogues ou les mélanges de celles-ci et un agent tensio-actif ou des mélanges d'agents tensio-actifs et sevelamer ou les pharmaceutiquement acceptables de celle-ci et facultativement un excipient ou des excipients supplémentaires.

7. Une seule dose unitaire de composition pharmaceutique selon les revendications 1 à 2 dans laquelle la seule dose unitaire est préparée sous forme de comprimé en comprimé où a) le noyau du comprimé ou l'externe du comprimé comprend une vitamine A, D, E ou K ou les analogues ou les mélanges de celles-ci et un agent tensio-actif ou des mélanges d'agents et facultativement excipient ou excîpîents supplémentaires ou b) le noyau du comprimé ou l'externe du comprimé comprend une vitamine A, D, E ou K ou les analogues ou les mélanges de celles-ci et un agent tensio-actif ou des mélanges d'agents tensio-actifs et une partie de sevelamer ou les sels pharmaceutiquement acceptables de celle-ci et facultativement excipient ou excipients supplémentaires ou, c) le noyau du comprimé ou l'externe du comprimé comprend un agent tensio-actif ou des mélanges d'agents tensio-actifs et sevelamer ou les sels pharmaceutiquement acceptables de celle-ci et facultativement excipient ou excipients supplémentaires, d) le noyau du comprimé ou l'externe du comprimé comprend une partie d'une vitamine A, D, E ou K ou les analogues ou les mélanges de celles-ci et un agent tensio-actif ou des mélanges d'agents tensio-actifs et une partie de sevelamer ou les sels pharmaceutiquement acceptables de celle-ci et facultativement excipient ou excipients supplémentaires.

8. Une seule dose unitaire de composition pharmaceutique selon les revendications 1 à 2 dans laquelle la seule dose unitaire est préparée sous forme d'un comprimé inlay où a) comprimé intérieur ou comprimé extérieur comprend une vitamine A, D, E ou K ou les analogues ou les mélanges de celles-ci et un agent tensio-actif ou des mélanges d'agents tensio-actifs et facultativement un excipient ou des excipients supplémentaires ou b)comprimé intérieur ou comprimé extérieur comprend une vitamine A, D, E ou K ou les analogues ou les mélanges de celles-ci et un agent tensio-actif ou des mélanges d'agents tensio-actifs et une partie de sevelamer ou les sels pharmaceutiquement acceptables de celle-ci et facultativement un excipient ou des excipients supplémentaires ou, c) comprimé intérieur ou comprimé extérieur comprend un agent tensio-actif ou des mélanges d'agents tensio-actifs et sevelamer ou les sels pharmaceutiquement acceptables de celle-ci et facultativement excipient ou excipients supplémentaires ou, d) comprimé intérieur ou comprimé extérieur comprend une partie de vitamine A, D, E ou K ou les analogues ou les mélanges de celles-ci et un agent tensio-actif ou des mélanges d'agents tensio-actifs et sevelamer ou les sels pharmaceutiquement acceptables de celle-ci et facultativement un excipient ou des excipients supplémentaires.

9. Une seule dose unitaire de composition pharmaceutique selon les revendications 1 à 2 dans laquelle la seule dose unitaire est préparée en granules ou poudres en sachet dans laquelle sevelamer ou les sels pharmaceutiquement acceptables de celle-ci et une vitamine choisie dans le groupe composé de vitamines A, D, E ou K ou les analogues ou les mélanges de celles-ci sont formulées conjointement avec un agent tensio-actif.

10. Une seule dose unitaire de composition pharmaceutique selon les revendications 1 à 9 l'agent tensio-actif utilisée ou des mélanges d'agents tensio-actifs, présente une valeur de balance hydrophile-lipophile de 0,5 à 40, préférablement de 5 à 20 et plus préférablement de 10 à 20.

11. Une seule dose unitaire de composition pharmaceutique selon les revendications précédents, en seule dose unitaire de compositions pharmaceutiques, de valeur d"acceptation de vitamine A, E, D ou K ou leurs analogues ou des mélanges de celles-ci n'est pas supérieur de 10,0, préférablement de la valeur d'acceptation n'est pas supérieur de 7,0, plus préférablement la valeur d'acceptation n'est pas supérieure de 6 et encore plus préférablement la valeur d'acceptation n'est pas supérieur à 5, en ce qui concerne l'uniformité du contenu.
